# EUROPEAN PATENT APPLICATION

(11) **EP 3 150 102 A1**
(43) Date of publication of application: **05.04.2017**
(21) Application number: 15799861.8
(22) Date of filing: 24.02.2015
(51) Int. Cl.: A61B 1/00, A61B 1/04, G02B 23/24, H04N 5/225

(54) **CAPSULE ENDOSCOPE APPARATUS**

(30) Priority: 27.05.2014 JP 2014109010
(71) Applicant: Olympus Corporation, Tokyo 192-8507 (JP)
(72) Inventor: MITSUHASHI, Kei, Tokyo 192-8507 (JP)
(74) Representative: Gunzelmann, Rainer
(86) International application number: PCT/JP2015/055202
(87) International publication number: WO 2015/182186

(57) **Abstract**

A small-sized capsule endoscope apparatus is provided. The capsule endoscope apparatus 2 is introduced into a subject and obtains in-vivo information on an inside of the subject and transmits the in-vivo information to an outside. The capsule endoscope apparatus 2 includes a shared coil 29 capable of transmitting and receiving radio waves and capable of implementing a plurality of functions, a function switching unit 27 configured to switch between functions of the shared coil 29, and a switching controller 28 configured to cause the function switching unit 27 to switch between the functions of the shared coil 29 at a predetermined timing.

## Description

### Field

The present invention relates to a capsule endoscope apparatus configured to be introduced into a subject and move inside a body cavity of the subject to obtain information on the subject.

### Background

In conventional endoscope fields, there is a known capsule endoscope apparatus that incorporates an imaging function, a wireless communication function, or the like, within a capsule-shaped casing having a size that can be introduced into a gastrointestinal tract of a subject such as a patient. This type of capsule endoscope apparatus is swallowed from the mouth of the subject, and thereafter, moved inside the subject, such as the gastrointestinal tract, with peristaltic action, or the like, and along with this movement, sequentially images portions inside the subject, generates image data, and sequentially transmits the captured image data wirelessly.

In order to achieve a small-sized capsule endoscope apparatus, there is a known technique that uses an AC starter coil instead of a reed switch as a power supply switch whose contact opens while being placed in a magnetic field (refer to Patent Literature 1).

### Citation List

### Patent Literature

Patent Literature 1: JP 2009-89907 A

### Summary

### Technical Problem

Unfortunately, however, the technique in the above-described Patent Literature 1 is required to arrange a wireless antenna for image transmission and an AC starter coil as the power switch separately from each other in the casing in order to avoid mutual interference of radio waves from the wireless antenna and the AC starter coil. Therefore, this configuration impedes the realization of a small-sized capsule endoscope apparatus.

The present invention has been made in view of the foregoing and an object of the present invention is to provide a small-sized capsule endoscope apparatus. Solution to Problem

In order to solve the above described problem and achieve the object, a capsule endoscope apparatus according to the invention is configured to be introduced into a subject, obtain in-vivo information on an inside of the subject, and transmit the obtained information outside the capsule endoscope apparatus. The capsule endoscope apparatus includes: a shared coil capable of transmitting and receiving radio waves and capable of implementing a plurality of functions; a function switching unit configured to switch between the functions of the shared coil; and a switching controller configured to cause the function switching unit to switch between the functions of the shared coil at a predetermined timing.

In the capsule endoscope apparatus according to the above-described invention, the plurality of functions includes two or more of: a transmitting function of transmitting the in-vivo information; a receiving function of receiving the radio waves from the outside to start or stop the capsule endoscope apparatus; a power supply function of receiving the radio waves from the outside to supply power to the capsule endoscope apparatus; and a position detection function of generating an alternate-current magnetic field to cause an external device to detect a position of the capsule endoscope apparatus.

The capsule endoscope apparatus according to the above-described invention further includes an imaging unit configured to image an imaging area to generate image data of the imaging area. The switching controller is configured to cause the function switching unit to switch between the functions of the shared coil based on a timing when the imaging unit generates the image data.

The capsule endoscope apparatus according to the above-described invention further includes a recording unit configured to record the image data generated by the imaging unit. The switching controller is configured to cause the function switching unit to switch between the functions of the shared coil based on a timing when the image data of a predetermined data amount is recorded on the recording unit.

In the capsule endoscope apparatus according to the above-described invention, the switching controller is configured to cause the function switching unit to sequentially switch between the functions of the shared coil for each one frame period in which the imaging unit generates the image data.

In the capsule endoscope apparatus according to the above-described invention, the imaging unit includes a plurality of pixels which is two-dimensionally arranged and each of which is configured to receive light and perform photoelectric conversion on the received light to generate the image data. The switching controller is configured to cause the function switching unit to sequentially switch between the functions of the shared coil for each one line period in which the image data is read from the pixels of one line on the imaging unit.

In the capsule endoscope apparatus according to the above-described invention, a length in time during which the shared coil is carrying out the receiving function in the one line period is longer than a sum of a length in time during which the shared coil is carrying out the transmitting function and a length in time from reception of the radio waves from outside until start or stop of the capsule endoscope apparatus.

In the capsule endoscope apparatus according to the above-described invention, the imaging unit includes a plurality of pixels which is two-dimensionally arranged and each of which is configured to receive light and perform photoelectric conversion on the received light to generate the image data. The switching controller is configured to cause the function switching unit to sequentially switch between the functions of the shared coil for each of predetermined lines on the imaging unit.

In the capsule endoscope apparatus according to the above-described invention, the imaging unit includes a first imaging unit and a second imaging unit configured to image different visual field areas.

### Advantageous Effects of Invention

Because the capsule endoscope apparatus according to the present invention performs transmission and reception of radio waves using a single shared coil, it is possible to achieve a smaller-sized capsule endoscope apparatus.

### Brief Description of Drawings

FIG. 1 is a schematic diagram illustrating an overview configuration of a capsule endoscope system according to a first embodiment of the present invention.
FIG. 2 is a block diagram illustrating a functional configuration of a capsule endoscope apparatus according to the first embodiment of the present invention.
FIG. 3 is a diagram illustrating a timing chart of operation executed by the capsule endoscope apparatus according to the first embodiment of the present invention.
FIG. 4 is a diagram illustrating a timing chart of operation executed by the capsule endoscope apparatus according to a second embodiment of the present invention.
FIG. 5 is a diagram illustrating a timing chart of operation executed by the capsule endoscope apparatus according to a third embodiment of the present invention.
FIG. 6 is a diagram illustrating a timing chart of operation executed by the capsule endoscope apparatus according to a fourth embodiment of the present invention.
FIG. 7 is a diagram illustrating a timing chart of operation executed by the capsule endoscope apparatus according to a fifth embodiment of the present invention.
FIG. 8 is a diagram illustrating a timing chart of operation executed by the capsule endoscope apparatus according to a sixth embodiment of the present invention.
FIG. 9 is a block diagram illustrating a functional configuration of the capsule endoscope apparatus according to a modification of the first to sixth embodiments of the present invention.

### Description of Embodiments

Hereinafter, modes for carrying out the present invention will be described in detail with reference to the drawings. Note that the present invention is not limited to the following embodiments. The drawings referred to in the following description merely schematically illustrate the shapes, sizes, and positional relations to such degrees that the contents of the present invention are understandable. Accordingly, the present invention is not limited only to the shapes, sizes, and positional relations exemplified in the individual drawings. The following description will exemplify a capsule endoscope system including a processing device that receives radio signals from a capsule endoscope apparatus configured to be introduced into a subject and to capture in-vivo images of the subject and that displays the in-vivo images of the subject. The present invention, however, is not limited to these embodiments. The same reference signs are used to designate the same elements throughout the drawings.

### (First Embodiment)

### [Overview Configuration of Capsule Endoscope System]

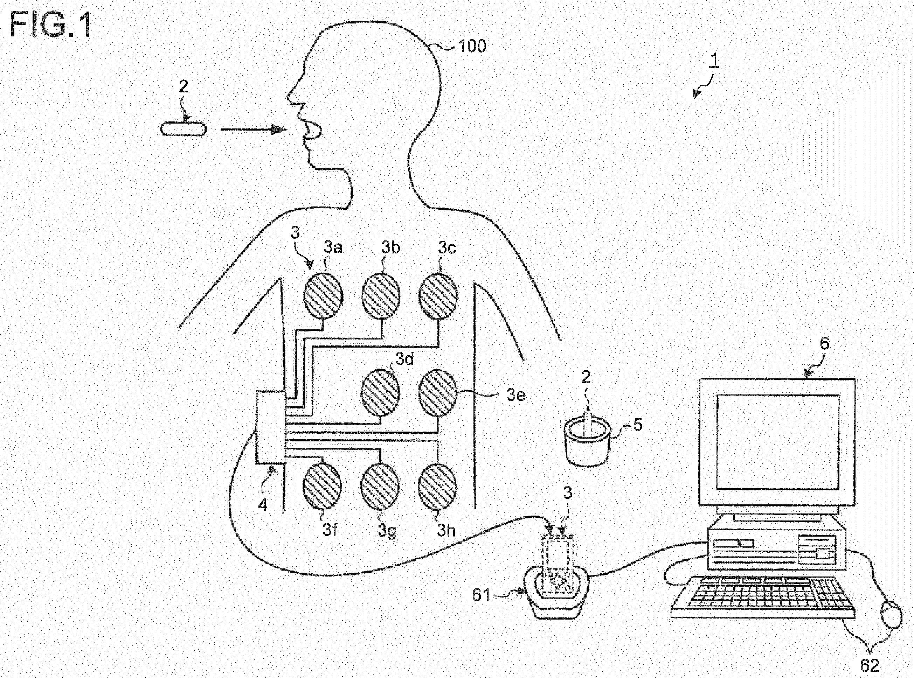

FIG. 1 is a schematic diagram illustrating an overview configuration of a capsule endoscope system according to a first embodiment of the present invention.

A capsule endoscope system 1 illustrated in FIG. 1 includes a capsule endoscope apparatus 2, a receiving antenna unit 3, a receiving device 4, a starter 5, and an image processing apparatus 6. The capsule endoscope apparatus 2 captures in-vivo images of a subject 100. The receiving antenna unit 3 receives radio signals transmitted from the capsule endoscope apparatus 2 introduced into the subject 100. The receiving device 4, while having the receiving antenna unit 3 removably connected to the device, performs predetermined processing on the radio signals received by the receiving antenna unit 3, and records or displays the radio signals. The starter 5 is configured to start or stop the capsule endoscope apparatus 2. The image processing apparatus 6 performs one or both of processing and displaying images corresponding to image data inside the subject 100 captured by the capsule endoscope apparatus 2.

The capsule endoscope apparatus 2 has an imaging function of imaging the inside of the subject 100 and a wireless communication function of transmitting in-vivo information including the image data obtained by imaging the inside of the subject 100 to the receiving antenna unit 3. The capsule endoscope apparatus 2 is swallowed into the subject 100, carried through the esophagus inside the subject 100, and moved inside the body cavity of the subject 100 by peristaltic action of the gastrointestinal lumen. The capsule endoscope apparatus 2, while moving inside the body cavity of the subject 100, sequentially images the inside of the body cavity of the subject 100 with a very short time interval, for example, 0.5 seconds, generates image data of the inside of the subject 100, and sequentially transmits the image data to the receiving antenna unit 3. A detailed configuration of the capsule endoscope apparatus 2 will be described below.

The receiving antenna unit 3 includes receiving antennas 3a to 3h. The receiving antennas 3a to 3h receive radio signals from the capsule endoscope apparatus 2 and transmit the radio signals to the receiving device 4. The receiving antennas 3a to 3h are configured to include loop antennas and arranged on predetermined positions on an external surface of the subject 100, for example, positions corresponding to individual organs inside the subject 100, namely, portions on a passage of the capsule endoscope apparatus 2.

The receiving device 4 records image data of the inside of the subject 100 included in the radio signals transmitted from the capsule endoscope apparatus 2 via the receiving antennas 3a to 3h, or displays an image corresponding to the image data of the inside of the subject 100. The receiving device 4 records positional information on the capsule endoscope apparatus 2, time information indicating time, or the like, in association with the image data received via the receiving antennas 3a to 3h. During examination by the capsule endoscope apparatus 2, specifically, during the period starting from a point where the device is introduced from the mouth of the subject 100, passing inside the gastrointestinal tract, until the device is discharged from the inside of the subject 100, the receiving device 4 is recorded in a receiving device holder (not illustrated) and carried by the subject 100. After completion of examination by the capsule endoscope apparatus 2, the receiving device 4 is removed from the subject 100 and then connected to the image processing apparatus 6 in order to transfer the image data, or the like, received from the capsule endoscope apparatus 2.

The starter 5 outputs a start signal to drive the capsule endoscope apparatus 2 and a stop signal to stop the capsule endoscope apparatus 2. Specifically, the starter 5 starts or stops the capsule endoscope apparatus 2 by generating a magnetic field. The starter 5 generates or stops the magnetic field by operating a switch (not illustrated).

The image processing apparatus 6 displays an image corresponding to the image data of the inside of the subject 100, obtained via the receiving device 4. The image processing apparatus 6 includes a cradle 61 that reads image data, or the like, from the receiving device 4, and an operation input device 62 such as a keyboard and a mouse. When the receiving device 4 is attached onto the cradle 61, the cradle 61 obtains, from the receiving device 4, image data and related information including positional information and time information associated with the image data, and identification information of the capsule endoscope apparatus 2, and transfers the various types of obtained information to the image processing apparatus 6. The operation input device 62 receives input from a user. While operating the operation input device 62, the user checks images of the inside of the subject 100, sequentially displayed by the image processing apparatus 6, and along with this, diagnoses the subject 100 by observing living sites inside the subject 100, for example, the esophagus, stomach, small intestine, and large intestine. The user further operates the operation input device 62 to generate a magnetic field on the starter 5, thereby starting or stopping the capsule endoscope apparatus 2.

### [Configuration of Capsule Endoscope Apparatus]

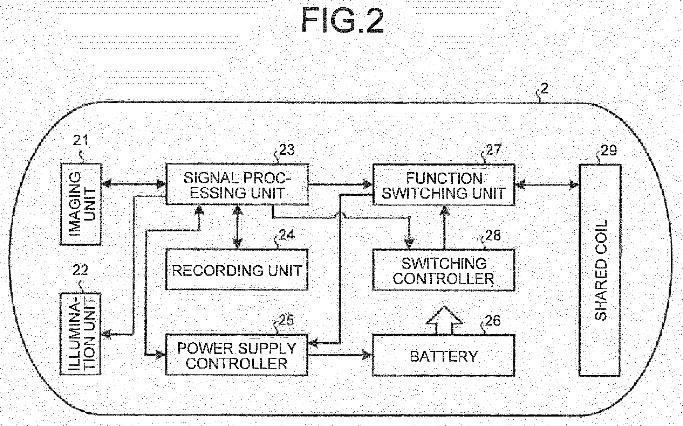

Next, a detailed configuration of the capsule endoscope apparatus 2 illustrated in FIG. 1 will be described. FIG. 2 is a block diagram illustrating a functional configuration of the capsule endoscope apparatus 2.

As illustrated in FIG. 2, the capsule endoscope apparatus 2 includes an imaging unit 21, an illumination unit 22, a signal processing unit 23, a recording unit 24, a power supply controller 25, a battery 26, a function switching unit 27, a switching controller 28, and a shared coil 29.

The imaging unit 21 includes an optical system that forms an optical image, and an imaging element. Exemplary imaging elements include a charge coupled device (CCD) and a complementary metal oxide semiconductor (CMOS). The imaging element includes a plurality of two-dimensionally arranged pixels configured to generate in-vivo image data of the subject 100 by receiving the optical image formed on a light receiving surface by the optical system and performing photoelectric conversion on the optical image. Under the control of the signal processing unit 23, the imaging unit 21 images an imaging area of the subject 100 at a predetermined frame rate, for example, 2 fps, and generates image data of the inside of the subject 100.

Under the control of the signal processing unit 23, the illumination unit 22 emits illumination light onto the inside of the subject 100 in synchronization with the frame rate of the imaging unit 21. The illumination unit 22 is formed with a light emitting diode (LED), or the like. The capsule endoscope apparatus 2 incorporates a circuit board (not illustrated) on which driving circuits, or the like, for separately driving the imaging unit 21 and the illumination unit 22. The imaging unit 21 and the illumination unit 22 are fixed on this circuit board, facing outward from one end of the capsule endoscope apparatus 2.

The signal processing unit 23 controls components of the capsule endoscope apparatus 2. The signal processing unit 23 generates digital image data by A/D converting analog image data output from the imaging unit 21 and performs predetermined signal processing on the image data.

The recording unit 24 temporarily records a program indicating various operations executed by the signal processing unit 23 and the image data that have undergone signal processing at the signal processing unit 23. The recording unit 24 records identification information for identifying the capsule endoscope apparatus 2.

The power supply controller 25 controls start or stop of the battery 26 based on a signal input from the function switching unit 27 described below.

The battery 26 supplies power to the components inside the capsule endoscope apparatus 2 under the control of the power supply controller 25. The battery 26 includes a primary battery or a secondary battery, such as a button battery, and a power supply circuit (not illustrated) that steps up the voltage of the power supplied from the button battery.

The function switching unit 27 switches between functions of the shared coil 29 under the control of the switching controller 28. The function switching unit 27 includes a plurality of circuits and causes the shared coil 29 to execute a plurality of predetermined functions by switching between the frequencies depending on whether the radio waves are to be transmitted or received, under the control of the switching controller 28. For example, when transmitting image data, the function switching unit 27 switches the function of the shared coil 29 to a receiving function by switching to a predetermined frequency.

The switching controller 28 causes the function switching unit 27 to switch between the functions of the shared coil 29 at a predetermined timing. Specifically, the switching controller 28 causes the function switching unit 27 to switch between the functions of the shared coil 29 based on the timing when the imaging unit 21 generates image data.

The shared coil 29 is capable of transmitting and receiving radio waves, and accordingly, capable of implementing a plurality of functions. Herein, the plurality of functions includes a transmitting function of transmitting image data (in-vivo information) to the outside, a receiving function of receiving radio waves from the starter 5 and starting or stopping the capsule endoscope apparatus 2, a power supply function of receiving radio waves from the outside and supplying power to the battery 26 of the capsule endoscope apparatus 2, and a position detection function of causing an external device to detect the position of the capsule endoscope apparatus 2. For example, the shared coil 29 either transmits image data input from the signal processing unit 23, or receives a start signal or a stop signal for the capsule endoscope apparatus 2 according to an AC magnetic field generated by the starter 5. In the first embodiment, the focus will be on the transmitting function and the receiving function of the shared coil 29 for the sake of simplification.

### [Operation of Capsule Endoscope Apparatus]

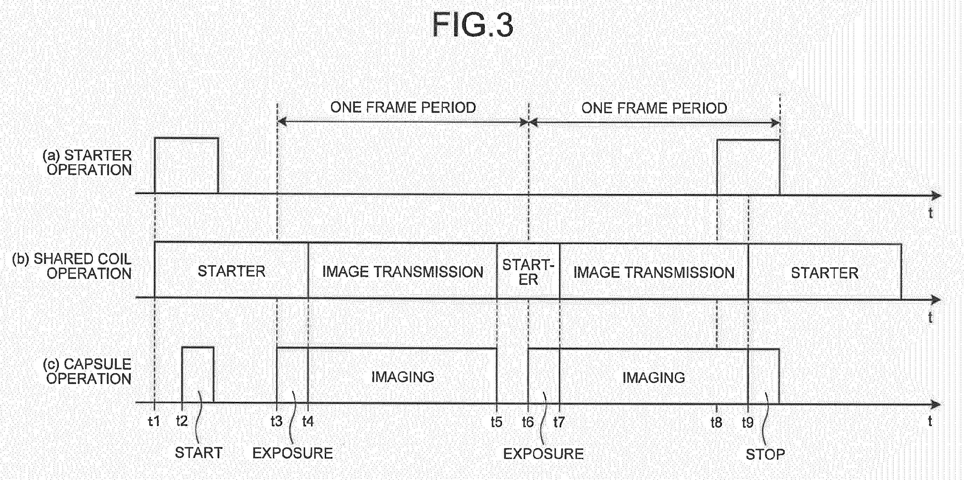

Next, operation executed by the capsule endoscope apparatus 2 will be descried. FIG. 3 is a diagram illustrating a timing chart of operation executed by the capsule endoscope apparatus 2. In FIG. 3, FIG. 3(a) illustrates timings of a start signal and a stop signal corresponding to an AC magnetic field output by the starter 5, FIG. 3(b) illustrates an operation timing of the shared coil 29, and FIG. 3(c) illustrates an operation timing of the capsule endoscope apparatus 2. In FIG. 3, the horizontal axis represents time.

First, as illustrated in FIG. 3, in a case where the function of the shared coil 29 is set to the receiving function and corresponds to a starter period (time t1 to time t4) in which signals are received from the starter 5, when a start signal is output from the starter 5 (time t1), the switching controller 28 outputs the start signal received by the shared coil 29 to the power supply controller 25. In response to this, the power supply controller 25 starts the battery 26 (time t2). As a result, the capsule endoscope apparatus 2 is started.

Subsequently, the imaging unit 21 starts exposure (time t3) in such a manner that the illumination unit 22 emits illumination light onto an imaging area and the reflected light is received by the imaging unit 21.

Thereafter, the imaging unit 21 starts imaging toward the imaging area. The switching controller 28 causes the function switching unit 27 to switch the function of the shared coil 29 to the transmitting function, and together with this, causes the shared coil 29 to transmit (time t4) the image data generated by the imaging unit 21.

Subsequently, after completion of transmission of the image data (time t5), the switching controller 28 causes the function switching unit 27 to switch (time t5) the function of the shared coil 29 to the receiving function (starter period). In this case, the imaging unit 21 starts exposure (time t6) in such a manner that the illumination unit 22 emits illumination light onto the imaging area and the reflected light is received by the imaging unit 21.

Thereafter, the imaging unit 21 starts imaging toward the imaging area. The switching controller 28 causes the function switching unit 27 to switch the function of the shared coil 29 to the transmitting function, and together with this, causes the shared coil 29 to transmit (time t7) the image data generated by the imaging unit 21. In this case, when the stop signal is output from the starter 5 (time t8), and after completion (time t9) of an image data transmission period (time t7 to time t9) by the shared coil 29, the switching controller 28 causes the function switching unit 27 to switch (time t9) the function of the shared coil 29 to the receiving function of receiving signals from the starter 5. At this time, the function switching unit 27 outputs the stop signal received by the shared coil 29 from the starter 5 to the power supply controller 25. In response to this, the power supply controller 25 stops the battery 26 (time t9). In this manner, by defining a period in which the imaging unit 21 is not performing imaging (e.g. time t5 to time t7) as a starter period (receiving function) for receiving signals from the starter 5 in one frame period, it is possible to provide the shared coil 29 having both the receiving function and the transmitting function. With this configuration, it is possible to decrease physical members of the capsule endoscope apparatus 2, and thus, to achieve a small-sized apparatus.

According to the first embodiment described above, the switching controller 28 switches between the functions of the function switching unit 27 based on a timing to generate image data by the imaging unit 21. This enables a single shared coil 29 to perform transmission of the image data and start or stop of the capsule endoscope apparatus 2, which makes it possible to achieve the small-sized capsule endoscope apparatus 2.

Furthermore, according to the first embodiment, the single shared coil 29 has both the receiving function and the transmitting function. This configuration can avoid mutual interference of the functions and can minimize constraints in arranging the capsule endoscope apparatus 2 inside the casing.

According to the first embodiment, the single shared coil 29 has both the receiving function and the transmitting function. This configuration can decrease the number of components and assembly steps for the capsule endoscope apparatus 2, and thus can reduce the cost.

In the first embodiment, provided that the starter 5 is within one frame period or within a starter period inside one frame, it is allowable to start or stop the capsule endoscope apparatus 2 by outputting a predetermined pattern, for example, a pulsed pattern that repeats turning on/off with a predetermined interval. With this configuration, it would be possible to avoid malfunction of the capsule endoscope apparatus 2.

### (Second Embodiment)

Next, a second embodiment of the present invention will be described. While having a configuration identical to the capsule endoscope apparatus of the above-described first embodiment, the capsule endoscope apparatus according to the second embodiment executes operation that differs from the capsule endoscope apparatus of the first embodiment. Specifically, the capsule endoscope apparatus 2 according to the first embodiment described above switches the functions of the shared coil 29 between the receiving function and the transmitting function in one frame period. In contrast, the capsule endoscope apparatus according to the second embodiment switches the functions of the shared coil 29 between the receiving function and the transmitting function in one line period. Accordingly, hereinafter, the focus will be on operation executed by the capsule endoscope apparatus according to the second embodiment. The same reference signs are used to designate the same elements as those of the capsule endoscope apparatus 2 according to the above-described first embodiment, and the explanation thereof will be omitted.

### [Operation of Capsule Endoscope Apparatus]

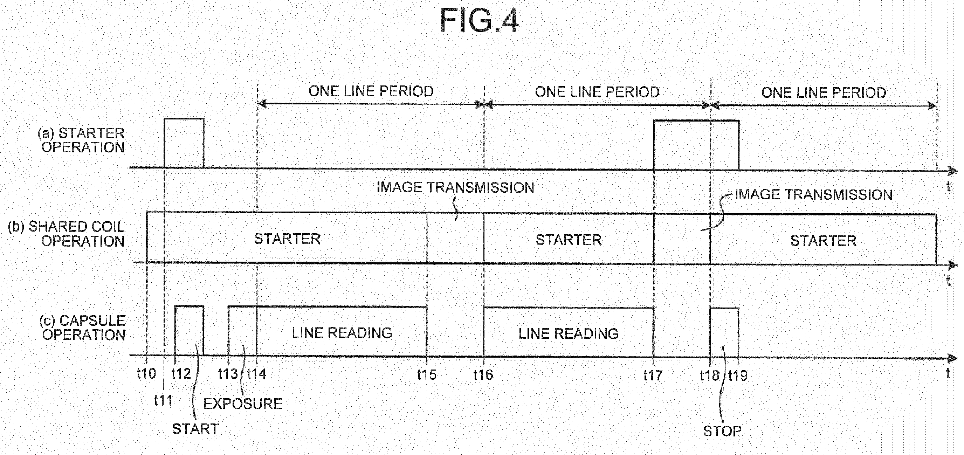

FIG. 4 is a diagram illustrating a timing chart of operation executed by the capsule endoscope apparatus 2 according to the second embodiment. In FIG. 4, FIG. 4(a) represents a timing of a start signal or a stop signal output by the starter 5, FIG. 4(b) represents an operation timing of the shared coil 29, and FIG. 4(c) represents an operation timing of the capsule endoscope apparatus 2. In FIG. 4, the horizontal axis represents time.

As illustrated in FIG. 4, in a case where the function of the shared coil 29 is set to the receiving function and to a starter period (time t10 to time t15), and when a start signal is output from the starter 5 (time t11), the function switching unit 27 outputs the start signal received by the shared coil 29 to the power supply controller 25. In response to this, the power supply controller 25 starts the battery 26 (time t12). As a result, the capsule endoscope apparatus 2 starts driving.

Subsequently, the imaging unit 21 starts exposure (time t13) in such a manner that the illumination unit 22 emits illumination light onto the imaging area and the reflected light is received by the imaging unit 21. Then, the imaging unit 21 performs line reading (time t14), namely, reading, per line, image data from pixels in one line. In this line reading period (time t14 to time t15), the switching controller 28 causes the function switching unit 27 to switch the function of the shared coil 29 to the transmitting function, and together with this, causes the shared coil 29 to transmit (time t15 to time t16) one-line image data read from the imaging unit 21.

Thereafter, the switching controller 28 causes the function switching unit 27 to switch (time t16 to time t17) the function of the shared coil 29 to the receiving function. In this starter period (reception period) (time t16 to time t17), the imaging unit 21 performs line reading of the image data from the next one line (time t16 to time t18).

Subsequently, the switching controller 28 causes the function switching unit 27 to switch the function of the shared coil 29 to the transmitting function, and together with this, causes the shared coil 29 to transmit (time t17 to time t18) one-line image data read from the imaging unit 21. When the stop signal is output from the starter 5 (time t17) in this image data transmission period (time t17 to time t18), the switching controller 28, after completion of transmission of one-line image data by the shared coil 29, causes the function switching unit 27 to switch (time t18) the function of the shared coil 29 to the receiving function. At this time, the function switching unit 27 outputs the stop signal received by the shared coil 29 from the starter 5 to the power supply controller 25. In response to this, the power supply controller 25 stops the battery 26. In this case, the switching controller 28 sets the time such that the length in time (time t16 to time t17) for which the function of the shared coil 29 for one line period is the receiving function is longer than the sum of the length in the transmitting function time (time t17 to time t18) for which the function of the shared coil 29 is to transmit the image, and the length in time (time t18 to time t19) from the reception of radio waves from outside until the start or stop of the capsule endoscope apparatus 2. With this setting, a user can start and stop the capsule endoscope apparatus 2 in any timing without concern for imaging timing at the imaging unit 21. In other words, the switching controller 28 sets the time of the receiving function to the time of a degree that cannot be recognized by the user.

According to the above-described second embodiment of the present invention, the switching controller 28 causes the function switching unit 27 to switch the functions of the shared coil 29 between the receiving function and the transmitting function in one line period in which image data is read from the imaging unit 21. Accordingly, the user can start and stop the capsule endoscope apparatus 2 in any timing.

### (Third Embodiment)

Next, a third embodiment of the present invention will be described. While having a configuration identical to the capsule endoscope apparatus of the above-described second embodiment, the capsule endoscope apparatus according to the third embodiment executes operation that differs from the capsule endoscope apparatus of the second embodiment. Specifically, the capsule endoscope apparatus 2 according to the above-described second embodiment transmits the one-line image data by the shared coil 29 immediately after the imaging unit 21 reads the data. In contrast, the capsule endoscope apparatus according to the third embodiment transmits data immediately after the one-line image data is temporarily recorded in the recording unit. Accordingly, hereinafter, the focus will be on operation executed by the capsule endoscope apparatus according to the third embodiment. The same reference signs are used to designate the same elements as those of the capsule endoscope apparatus 2 according to the above-described first embodiment, and the explanation thereof will be omitted.

### [Operation of Capsule Endoscope Apparatus]

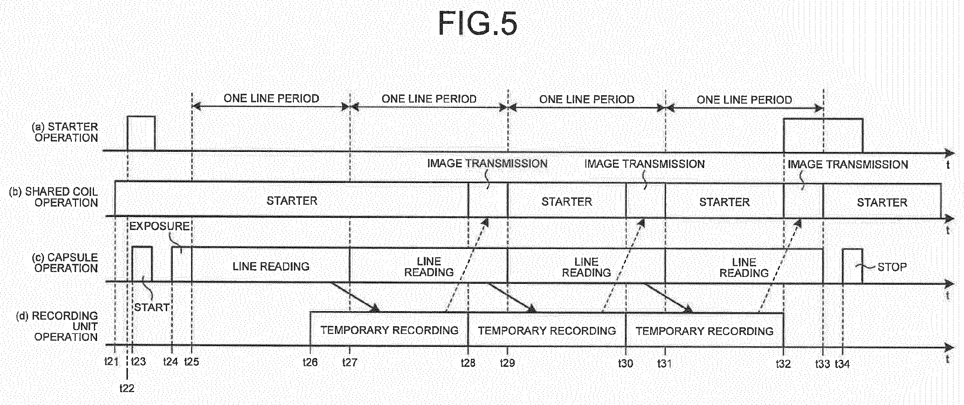

FIG. 5 is a diagram illustrating a timing chart of operation executed by the capsule endoscope apparatus 2 according to the third embodiment. In FIG. 5, FIG. 5(a) represents a timing of a start signal or a stop signal output by the starter 5, FIG. 5(b) represents an operation timing of the shared coil 29, FIG. 5(c) represents an operation timing of the capsule endoscope apparatus 2, and FIG. 5(d) represents a recording timing of the recording unit 24. In FIG. 5, the horizontal axis represents time.

As illustrated in FIG. 5, in a case where the function of the shared coil 29 is set to the receiving function and to a starter period (time t21 to time t28), and when a start signal is output from the starter 5 (time t22), the function switching unit 27 outputs the start signal received by the shared coil 29 to the power supply controller 25. In response to this, the power supply controller 25 starts the battery 26 (time t23). As a result, the capsule endoscope apparatus 2 starts driving.

Subsequently, the imaging unit 21 starts exposure (time t24) in such a manner that the illumination unit 22 emits illumination light onto the imaging area and the reflected light is received by the imaging unit 21. The imaging unit 21 starts line reading (time t25), namely, reading image data from charged pixels, per line.

Thereafter, the signal processing unit 23 starts (time t26) temporary recording, namely, temporarily recording one-line image data read from the imaging unit 21, onto the recording unit 24.

Subsequently, the imaging unit 21 starts line reading (time t27) of the image data from the next one line.

Thereafter, when the image data of a data amount of one line is temporarily recorded in the recording unit 24 (time t28), the switching controller 28 causes the function switching unit 27 to switch the function of the shared coil 29 to the transmitting function, and together with this, causes the shared coil 29 to transmit (time t28) image data temporarily recorded in the recording unit 24 (one-line image data read from time t25 to time t27).

Subsequently, the switching controller 28 causes the function switching unit 27 to switch (time t29) the function of the shared coil 29 to the receiving function, and the imaging unit 21 performs line reading of the image data from the next one line (time t29). Together with this, the signal processing unit 23 starts temporary recording (time t28), that is, temporarily recording one-line image data read from the imaging unit 21 (one-line image data read from time t27 to time t29) onto the recording unit 24.

Thereafter, when temporarily recording of the one-line image data onto the recording unit 24 is completed (time t30), the switching controller 28 causes the function switching unit 27 to switch the function of the shared coil 29 to the transmitting function, and together with this, causes the shared coil 29 to transmit (time t30) image data temporarily recorded in the recording unit 24 (one-line image data read from time t27 to time t29).

Subsequently, the switching controller 28 causes the function switching unit 27 to switch the function of the shared coil 29 to the receiving function, and the imaging unit 21 performs line reading of the image data from the next one line. Together with this, the signal processing unit 23 starts temporary recording (time t31), that is temporarily recording one-line image data read from the imaging unit 21 onto the recording unit 24.

Subsequently, when temporary recording of the one-line image data onto the recording unit 24 is completed (time t32), the switching controller 28 causes the function switching unit 27 to switch the function of the shared coil 29 to the transmitting function, and together with this, causes the shared coil 29 to transmit (time t32) the image data temporarily recorded in the recording unit 24 (one-line image data read from time t29 to time t31). In a case where a stop signal is output from the starter 5 during the image data transmission period (time t32 to time t33), the shared coil 29, since it has not been switched to the receiving function, cannot receive the stop signal from the starter 5.

Thereafter, the switching controller 28, after transferring the image data (time t33), causes the function switching unit 27 to switch the functions of the shared coil 29 to the receiving function. In this case, the function switching unit 27 outputs the stop signal received by the shared coil 29 to the power supply controller 25. In response to this, the power supply controller 25 stops the battery 26 (time t34).

According to the third embodiment described above, even in a period when one-line image data is being read from the imaging unit 21, it is possible to perform transmission of image data and start/stop of the capsule endoscope apparatus 2 with one shared coil 29. As a result, it is possible to achieve the smaller-sized capsule endoscope apparatus 2.

In the third embodiment, the function of the function switching unit 27 is switched between the receiving function and the transmitting function for one line period. Alternatively, it is also possible to switch between the receiving function and the transmitting function for one frame period.

### (Fourth Embodiment)

Next, a fourth embodiment of the present invention will be described. While having a configuration identical to the capsule endoscope apparatus of the above-described second embodiment, the capsule endoscope apparatus according to the fourth embodiment executes operation that differs from the capsule endoscope apparatus of the second embodiment. Specifically, the capsule endoscope apparatus according to the fourth embodiment sequentially switches the function of the shared coil, for one line period, to any of the position detection function, the power supply function, the receiving function, and the transmitting function. Accordingly, hereinafter, the focus will be on operation executed by the capsule endoscope apparatus according to the fourth embodiment. The same reference signs are used to designate the same elements as those of the capsule endoscope apparatus 2 according to the above-described first embodiment, and the explanation thereof will be omitted.

### [Operation of Capsule Endoscope Apparatus]

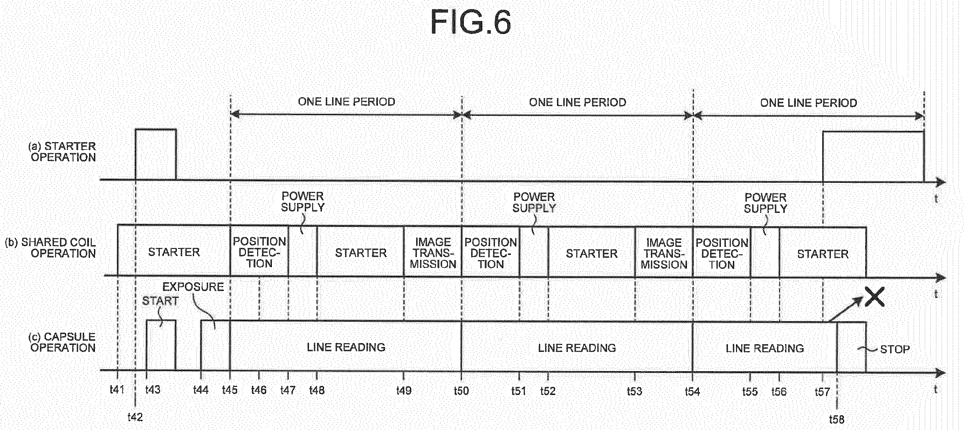

FIG. 6 is a diagram illustrating a timing chart of operation executed by the capsule endoscope apparatus 2 according to the fourth embodiment. In FIG. 6, FIG. 6(a) represents a timing of a start signal or a stop signal output by the starter 5, FIG. 6(b) represents an operation timing of the shared coil 29, and FIG. 6(c) represents an operation timing of the capsule endoscope apparatus 2. In FIG. 6, the horizontal axis represents time.

As illustrated in FIG. 6, in a case where the function of the shared coil 29 is set to the receiving function and to a starter period (time t41 to time t45), and when a start signal is output from the starter 5 (time t42), the function switching unit 27 outputs the start signal received by the shared coil 29 to the power supply controller 25. In response to this, the power supply controller 25 starts the battery 26 (time t43). As a result, the capsule endoscope apparatus 2 starts driving.

Subsequently, the imaging unit 21 starts exposure (time t44) in such a manner that the illumination unit 22 emits illumination light onto an imaging area, and the reflected light is received by the imaging unit 21.

Thereafter, the switching controller 28 causes the function switching unit 27 to switch (time t45) the function of the shared coil 29 to the position detection function for detecting the position of the capsule endoscope apparatus 2. Specifically, the power supply controller 25 causes an external position detection device to detect the position by generating an AC magnetic field by applying current to the shared coil 29 from the battery 26 via the function switching unit 27. At this time, the imaging unit 21 performs line reading, namely, reading, per line, image data from pixels in one line (time t45).

Subsequently, the switching controller 28 causes the function switching unit 27 to switch (time t47) the function of the shared coil 29 to the power supply function of supplying power to the battery 26 by converting the radio waves transmitted from the outside to power.

Thereafter, the switching controller 28 causes the function switching unit 27 to switch (time t48) the function of the shared coil 29 to the receiving function (starter period).

Subsequently, the switching controller 28 causes the function switching unit 27 to switch the function of the shared coil 29 to the transmitting function, and starts transmission of the one-line image data read from the imaging unit 21 (time t49).

Thereafter, the switching controller 28 causes the function switching unit 27 to switch (time t50) the function of the shared coil 29 to the position detection function. At this time, the imaging unit 21 starts line reading (time t50) of the image data from the next one line.

Subsequently, the switching controller 28 causes the function switching unit 27 to switch (time t51) the function of the shared coil 29 to the power supply function.

Thereafter, the switching controller 28 causes the function switching unit 27 to switch the function of the shared coil 29 to the receiving function (time t52).

Subsequently, the switching controller 28 causes the function switching unit 27 to switch the function of the shared coil 29 to the transmitting function, and starts transmission of the one-line image data read from the imaging unit 21 (time t53).

Thereafter, the switching controller 28 causes the function switching unit 27 to switch (time t54) the function of the shared coil 29 to the position detection function. At this time, the imaging unit 21 starts line reading (time t54) of the image data from the next one line.

Subsequently, the switching controller 28 causes the function switching unit 27 to switch the function of the shared coil 29 to the power supply function (time t55).

Thereafter, the switching controller 28 causes the function switching unit 27 to switch (time t56) the function of the shared coil 29 to the receiving function. In a case where the stop signal is output from the starter 5 in this starter period (time t57), the stop signal received by the shared coil 29 is output onto the power supply controller 25. In response to this, the power supply controller 25 stops the battery 26 (time t58).

According to the fourth embodiment described above, it is possible to provide one shared coil 29 with the receiving function, the transmitting function, the power supply function, and the position detection function. Accordingly, it is possible to achieve the smaller-sized capsule endoscope apparatus 2.

### (Fifth Embodiment)

Next, a fifth embodiment of the present invention will be described. While having a configuration identical to the capsule endoscope apparatus of the above-described first embodiment, the capsule endoscope apparatus according to the fifth embodiment executes operation that differs from the capsule endoscope apparatus of the first embodiment. Specifically, the capsule endoscope apparatus according to the fifth embodiment sequentially switches the function of the shared coil, for a predetermined line on the imaging unit, to any of the position detection function, the power supply function, the receiving function, and the transmitting function. Accordingly, hereinafter, the focus will be on operation executed by the capsule endoscope apparatus according to the fifth embodiment. The same reference signs are used to designate the same elements as those of the capsule endoscope apparatus 2 according to the above-described first embodiment, and the explanation thereof will be omitted.

### [Operation of Capsule Endoscope Apparatus]

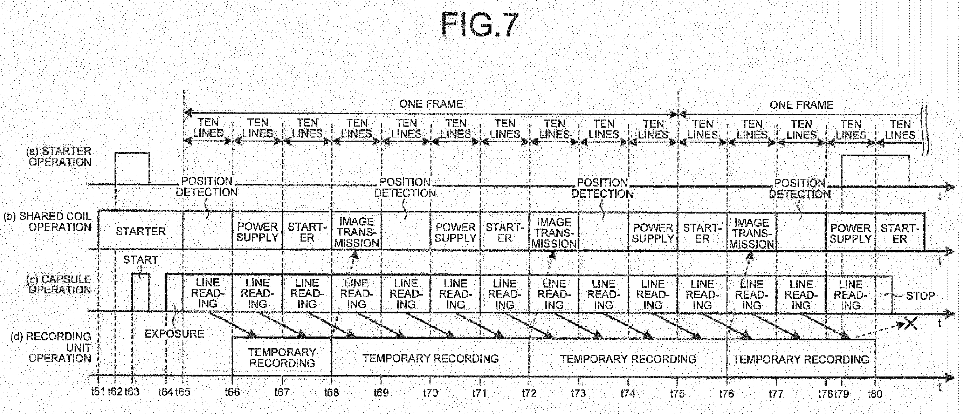

FIG. 7 is a diagram illustrating a timing chart of operation executed by the capsule endoscope apparatus 2 according to the fifth embodiment. In FIG. 7, FIG. 7(a) represents a timing of a start signal or a stop signal output by the starter 5, FIG. 7(b) represents an operation timing of the shared coil 29, FIG. 7(c) represents an operation timing of the capsule endoscope apparatus 2, and FIG. 7(d) represents a recording timing of the recording unit 24. In FIG. 7, the horizontal axis represents time.

As illustrated in FIG. 7, in a case where the function of the shared coil 29 is set to the receiving function and to a starter period (time t61 to time t65), and when a start signal is output from the starter 5 (time t62), the function switching unit 27 outputs the start signal received by the shared coil 29 to the power supply controller 25. In response to this, the power supply controller 25 starts the battery 26 (time t63). As a result, the capsule endoscope apparatus 2 starts driving.

Subsequently, the imaging unit 21 starts exposure (time t64) in such a manner that the illumination unit 22 emits illumination light onto an imaging area and the reflected light is received by the imaging unit 21.

Thereafter, the switching controller 28 causes the function switching unit 27 to switch (time t65) the function of the shared coil 29 to the position detection function for detecting the position of the capsule endoscope apparatus 2. Specifically, the power supply controller 25 causes an external position detection device to detect the position by generating an AC magnetic field by applying current to the shared coil 29 from the battery 26 via the function switching unit 27. At this time, the imaging unit 21 performs line reading, namely, reading, per line, image data from pixels in one line (time t65).

Subsequently, after a predetermined number of lines, for example, 10 lines, read from the imaging unit 21, have elapsed, the switching controller 28 causes the function switching unit 27 to switch (time t66) the function of the shared coil 29 to the power supply function of supplying power to the battery 26 by converting the radio waves transmitted from the outside to power. In this case, the signal processing unit 23 starts (time t66) temporary recording, namely, temporarily recording image data for 10 lines read from the imaging unit 21.

Then, after 10 lines read from the imaging unit 21 have elapsed, the switching controller 28 causes the function switching unit 27 to switch (time t67) the function of the shared coil 29 to the receiving function (starter period).

Subsequently, after 10 lines read from the imaging unit 21 have elapsed, the switching controller 28 causes the function switching unit 27 to switch the function of the shared coil 29 to the transmitting function, and together with this, starts transmission (time t68) of the image data (image data read from time t66 to time t67) temporarily recorded in the recording unit 24. In this case, the signal processing unit 23 starts (time t68) temporary recording, namely, temporarily recording image data (image data from time t67 to time t68) for 10 lines read from the imaging unit 21.

Then, after 10 lines read from the imaging unit 21 have elapsed, the switching controller 28 causes the function switching unit 27 to switch (time t69) the function of the shared coil 29 to the position detection function.

Subsequently, after 10 lines read from the imaging unit 21 have elapsed, the switching controller 28 causes the function switching unit 27 to switch (time t70) the function of the shared coil 29 to the power supply function.

Then, after 10 lines read from the imaging unit 21 have elapsed, the switching controller 28 causes the function switching unit 27 to switch (time t71) the function of the shared coil 29 to the receiving function.

Subsequently, after 10 lines read from the imaging unit 21 have elapsed, the switching controller 28 causes the function switching unit 27 to switch the function of the shared coil 29 to the transmitting function, and together with this, starts transmission (time t72) of the image data (image data read from time t67 to time t70) temporarily recorded in the recording unit 24. In this case, the signal processing unit 23 starts (time t72) temporary recording, namely, temporarily recording image data (image data from time t71 to time t72) for 10 lines read from the imaging unit 21.

Then, after 10 lines read from the imaging unit 21 have elapsed, the switching controller 28 causes the function switching unit 27 to switch (time t73) the function of the shared coil 29 to the position detection function.

Subsequently, after 10 lines read from the imaging unit 21 have elapsed, the switching controller 28 causes the function switching unit 27 to switch (time t74) the function of the shared coil 29 to the power supply function.

Then, after 10 lines read from the imaging unit 21 have elapsed, the switching controller 28 causes the function switching unit 27 to switch (time t75) the function of the shared coil 29 to the receiving function.

Subsequently, after 10 lines read from the imaging unit 21 have elapsed, the switching controller 28 causes the function switching unit 27 to switch the function of the shared coil 29 to the transmitting function, and together with this, starts transmission (time t76) of the image data (image data read from time t71 to time t74) temporarily recorded in the recording unit 24. In this case, the signal processing unit 23 starts (time t76) temporary recording, namely, temporarily recording image data (image data from time t75 to time t76) for 10 lines read from the imaging unit 21.

Then, after 10 lines read from the imaging unit 21 have elapsed, the switching controller 28 causes the function switching unit 27 to switch (time t77) the function of the shared coil 29 to the position detection function.

Subsequently, after 10 lines read from the imaging unit 21 have elapsed, the switching controller 28 causes the function switching unit 27 to switch (time t78) the function of the shared coil 29 to the power supply function.

Then, after 10 lines read from the imaging unit 21 have elapsed, the switching controller 28 causes the function switching unit 27 to switch (time t80) the function of the shared coil 29 to the receiving function. In a case where the stop signal is output from the starter 5 in this starter period (time t79), the stop signal received by the shared coil 29 is output onto the power supply controller 25. In response to this, the power supply controller 25 stops the battery 26 (time t80).

According to the fifth embodiment described above, it is possible with one shared coil 29 to switch among the receiving function, the transmitting function, the power supply function, and the position detection function, for each of predetermined lines of the imaging unit 21. Accordingly, it is possible to achieve the smaller-sized capsule endoscope apparatus 2.

In the above-described fifth embodiment, the switching controller 28 switches the function of the shared coil 29 among the receiving function, the transmitting function, the power supply function, and the position detection function, for every 10 lines of the imaging unit 21. Alternatively, however, the number of lines for switching can be set as appropriate. Specifically, it is possible to switch the function of the shared coil 29, for example, for every five, 20, or 25 lines.

### (Sixth Embodiment)

Next, a sixth embodiment of the present invention will be described. While having a configuration identical to the capsule endoscope apparatus of the above-described first embodiment, the capsule endoscope apparatus according to the sixth embodiment executes operation that differs from the capsule endoscope apparatus of the first embodiment. Specifically, the capsule endoscope apparatus according to the sixth embodiment sequentially switches the function of the shared coil, for one frame period of the imaging unit, to any of the position detection function, the power supply function, the receiving function, and the transmitting function. Accordingly, hereinafter, the focus will be on operation executed by the capsule endoscope apparatus according to the sixth embodiment. The same reference signs are used to designate the same elements as those of the capsule endoscope apparatus 2 according to the above-described first embodiment, and the explanation thereof will be omitted.

### [Operation of Capsule Endoscope Apparatus]

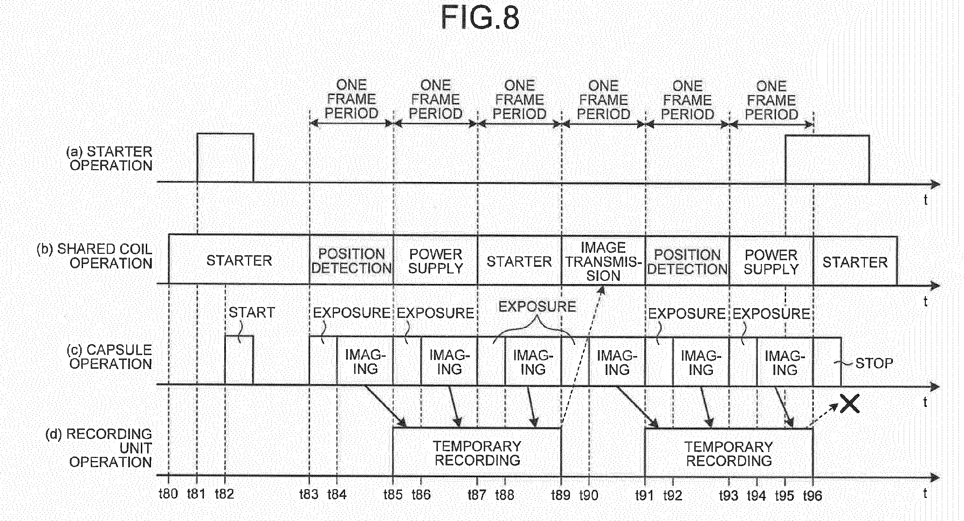

FIG. 8 is a diagram illustrating a timing chart of operation executed by the capsule endoscope apparatus 2 according to the sixth embodiment of the present invention. In FIG. 8, FIG. 8(a) represents a timing of a start signal or a stop signal output by the starter 5, FIG. 8(b) represents an operation timing of the shared coil 29, FIG. 8(c) represents an operation timing of the capsule endoscope apparatus 2, and FIG. 8(d) represents a recording timing of the recording unit 24. In FIG. 8, the horizontal axis represents time.

As illustrated in FIG. 8, in a case where the function of the shared coil 29 is set to the receiving function and to a starter period (time t80 to time t83) in which signals are received from the starter 5, and when a start signal is output from the starter 5 (time t81), the switching controller 28 outputs the start signal received by the shared coil 29 to the power supply controller 25. In response to this, the power supply controller 25 starts the battery 26 (time t82). As a result, the capsule endoscope apparatus 2 is started.

Subsequently, the imaging unit 21 starts exposure in such a manner that the illumination unit 22 emits illumination light onto an imaging area and the reflected light is received by the imaging unit 21. The switching controller 28 causes the function switching unit 27 to switch (time t83) the function of the shared coil 29 to the position detection function. Thereafter, the imaging unit 21 starts imaging toward the imaging area (time t84).

Subsequently, the imaging unit 21 starts exposure in such a manner that the illumination unit 22 emits illumination light onto an imaging area and the reflected light is received by the imaging unit 21. The switching controller 28 causes the function switching unit 27 to switch (time t85) the function of the shared coil 29 to the power supply function. In this case, the imaging unit 21 starts exposure in such a manner that the illumination unit 22 emits illumination light onto an imaging area and the reflected light is received by the imaging unit 21. Together with this, the signal processing unit 23 starts (time t85) temporary recording, namely, temporarily recording image data for one frame read from the imaging unit 21. Thereafter, the imaging unit 21 starts imaging (time t86).

Subsequently, the switching controller 28 causes the function switching unit 27 to switch the function of the shared coil 29 to the receiving function. Together with this, the imaging unit 21 starts exposure (time t87) in such a manner that the illumination unit 22 emits illumination light onto the imaging area and the reflected light is received by the imaging unit 21. Thereafter, the imaging unit 21 starts imaging toward the imaging area (time t88).

Subsequently, the imaging unit 21 starts exposure in such a manner that the illumination unit 22 emits illumination light onto an imaging area and the reflected light is received by the imaging unit 21. The switching controller 28 causes the function switching unit 27 to switch (time t89) the function of the shared coil 29 to the transmitting function. In this case, the shared coil 29 starts transmission (time t89) of the image data (image data read from time t85 to time t89) temporarily recorded in the recording unit 24. Thereafter, the imaging unit 21 starts imaging toward the imaging area (time t90).

Subsequently, the imaging unit 21 starts exposure in such a manner that the illumination unit 22 emits illumination light onto an imaging area and the reflected light is received by the imaging unit 21. The switching controller 28 causes the function switching unit 27 to switch (time t91) the function of the shared coil 29 to the position detection function. In this case, the signal processing unit 23 starts (time t91) temporary recording, namely, temporarily recording image data read from the imaging unit 21. Thereafter, the imaging unit 21 starts imaging toward the imaging area (time t92).

Subsequently, the imaging unit 21 starts exposure in such a manner that the illumination unit 22 emits illumination light onto an imaging area and the reflected light is received by the imaging unit 21. The switching controller 28 causes the function switching unit 27 to switch (time t93) the function of the shared coil 29 to the power supply function. Thereafter, the imaging unit 21 starts imaging toward the imaging area (time t94). In this case, when a stop signal is output from the starter 5 (time t95), and when the function of the shared coil 29 has been switched to the receiving function by the switching controller 28 via the function switching unit 27 (time t96), the function switching unit 27 outputs the stop signal received by the shared coil 29 from the starter 5 to the power supply controller 25. In response to this, the power supply controller 25 stops the battery 26 (time t96).

According to the sixth embodiment described above, switching controller 28 switches the function of one shared coil 29 among the receiving function, the transmitting function, the power supply function, and the position detection function, for one frame period of the imaging unit 21. Accordingly, it is possible to achieve the smaller-sized capsule endoscope apparatus 2.

In the above-described sixth embodiment, the switching controller 28 switches the function of the shared coil 29 among the receiving function, the transmitting function, the power supply function, and the position detection function, for one frame period of the imaging unit 21. Alternatively, the frame period for switching can be set as appropriate. Specifically, it is possible to switch the function of the shared coil 29, for example, for every two frame periods or five fame periods.

### (Modification)

Next, a modification of the first to sixth embodiments of the present invention will be described. The capsule endoscope apparatus 2 according to the above-described first to sixth embodiments has a configuration for imaging a single imaging area (monocular system). In contrast, a capsule endoscope apparatus according to the first to sixth embodiments can image different visual field areas (binocular system). Accordingly, the same reference signs are used to designate the same elements as those of the capsule endoscope apparatus 2 according to the above-described first to sixth embodiments, and the explanation thereof will be omitted.

### [Configuration of Capsule Endoscope]

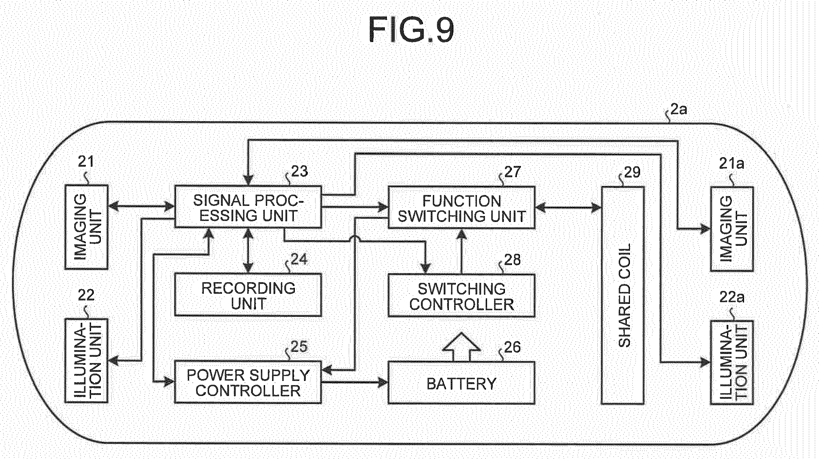

FIG. 9 is a block diagram illustrating a functional configuration of the capsule endoscope apparatus according to the modification of the first to sixth embodiments.

In addition to the configuration of the capsule endoscope apparatus 2 according to the above-described first to sixth embodiments, a capsule endoscope apparatus 2a illustrated in FIG. 9 includes an imaging unit 21a and an illumination unit 22a.

The imaging unit 21a has a configuration similar to the configuration of the above-described imaging unit 21. Under control of the signal processing unit 23, the imaging unit 21a generates in-vivo image data of the subject 100, and outputs the in-vivo image data to the signal processing unit 23.

The illumination unit 22a has a configuration similar to the configuration of the above-described illumination unit 22. Under the control of the signal processing unit 23, the illumination unit 22a emits illumination light onto the inside of the subject 100 in synchronization with the frame rate of the imaging unit 21a.

According to the above-described modification of the first to sixth embodiments, the capsule endoscope apparatus 2a further includes the imaging unit 21a and the illumination unit 22a inside the casing. With this configuration, it is possible to achieve a smaller-sized apparatus by providing the single shared coil 29 with a plurality of functions even if physical space inside the casing is restricted.

In this manner, the present invention in its broader aspects is not limited to the representative embodiments described herein. Accordingly, various modifications can be made without departing from the spirit or scope of the general inventive concept as defined by the appended claims and their equivalents.

### Reference Signs List

- 1: CAPSULE ENDOSCOPE SYSTEM
- 2, 2a: CAPSULE ENDOSCOPE APPARATUS
- 3: RECEIVING ANTENNA UNIT
- 3a to 3h: RECEIVING ANTENNA
- 4: RECEIVING DEVICE
- 5: STARTER
- 6: IMAGE PROCESSING APPARATUS
- 21, 21a: IMAGING UNIT
- 22, 22a: ILLUMINATION UNIT
- 23: SIGNAL PROCESSING UNIT
- 24: RECORDING UNIT
- 25: POWER SUPPLY CONTROLLER
- 26: BATTERY
- 27: FUNCTION SWITCHING UNIT
- 28: SWITCHING CONTROLLER
- 29: SHARED COIL
- 61: CRADLE
- 62: OPERATION INPUT DEVICE
- 100: SUBJECT

## Claims

1. A capsule endoscope apparatus configured to be introduced into a subject, obtain in-vivo information on an inside of the subject, and transmit the obtained information outside the capsule endoscope apparatus, the capsule endoscope apparatus comprising:
a shared coil capable of transmitting and receiving radio waves and capable of implementing a plurality of functions;
a function switching unit configured to switch between the functions of the shared coil; and
a switching controller configured to cause the function switching unit to switch between the functions of the shared coil at a predetermined timing.

2. The capsule endoscope apparatus according to claim 1, wherein the plurality of functions includes two or more of:
a transmitting function of transmitting the in-vivo information; a receiving function of receiving the radio waves from the outside to start or stop the capsule endoscope apparatus; a power supply function of receiving the radio waves from the outside to supply power to the capsule endoscope apparatus; and a position detection function of generating an alternate-current magnetic field to cause an external device to detect a position of the capsule endoscope apparatus.

3. The capsule endoscope apparatus according to claim 2, further comprising an imaging unit configured to image an imaging area to generate image data of the imaging area, wherein
the switching controller is configured to cause the function switching unit to switch between the functions of the shared coil based on a timing when the imaging unit generates the image data.

4. The capsule endoscope apparatus according to claim 3, further comprising a recording unit configured to record the image data generated by the imaging unit, wherein
the switching controller is configured to cause the function switching unit to switch between the functions of the shared coil based on a timing when the image data of a predetermined data amount is recorded on the recording unit.

5. The capsule endoscope apparatus according to claim 3, wherein the switching controller is configured to cause the function switching unit to sequentially switch between the functions of the shared coil for each one frame period in which the imaging unit generates the image data.

6. The capsule endoscope apparatus according to claim 3, wherein
the imaging unit includes a plurality of pixels which is two-dimensionally arranged and each of which is configured to receive light and perform photoelectric conversion on the received light to generate the image data, and
the switching controller is configured to cause the function switching unit to sequentially switch between the functions of the shared coil for each one line period in which the image data is read from the pixels of one line on the imaging unit.

7. The capsule endoscope apparatus according to claim 6, wherein
a length in time during which the shared coil is carrying out the receiving function in the one line period is longer than a sum of a length in time during which the shared coil is carrying out the transmitting function and a length in time from reception of the radio waves from outside until start or stop of the capsule endoscope apparatus.

8. The capsule endoscope apparatus according to claim 3, wherein
the imaging unit includes a plurality of pixels which is two-dimensionally arranged and each of which is configured to receive light and perform photoelectric conversion on the received light to generate the image data, and
the switching controller is configured to cause the function switching unit to sequentially switch between the functions of the shared coil for each of predetermined lines on the imaging unit.

9. The capsule endoscope apparatus according to claim 3, wherein the imaging unit includes a first imaging unit and a second imaging unit configured to image different visual field areas.
